# EUROPEAN PATENT SPECIFICATION

(11) **EP 2 205 175 B1**
(45) Date of publication and mention of the grant of the patent: **27.01.2016**
(21) Application number: 08843029.3
(22) Date of filing: 24.10.2008
(51) Int. Cl.: A61B 17/00, A61B 18/00, A61F 9/008, A61B 18/22

(54) **SURGICAL HANDPIECE FOR ENDOCANALICULAR LASER DACRYOCYSTORHINOSTOMY**
CHIRURGISCHES HANDSTÜCK FÜR ENDOKANALIKULÄRE LASER-DAKRYOZYSTORHINOSTOMIE
PIÈCE À MAIN CHIRURGICALE POUR DACRYOCYSTORHINOSTOMIE AU LASER PAR VOIE ENDOCANALICULAIRE

(30) Priority: 26.10.2007 SI 200700278
(43) Date of publication of application: 14.07.2010
(73) Proprietor: Optotek d.o.o., 1000 Ljubljana (SI)
(72) Inventor: DRNOVSEK-OLUP Brigita, 1000 Ljubljana (SI); LAMOT, Kristijan, 3205 Vitanje (SI)
(74) Representative: Flak, Antonija
(86) International application number: PCT/SI2008/000056
(87) International publication number: WO 2009/054816

(56) References cited:
- WO-A-2004/091380
- WO-A-2005/048817
- WO-A-2007/021914
- WO-A-2007/053590
- US-A- 6 159 207
- US-A1- 2002 151 879

## Description

### Field of the invention

This invention relates generally to medical devices for eye surgery, and more particularly to medical devices for endocanalicular dacryocystorhinostomy.

### Description of the technical problem

The technical problem to be solved by the present invention is to provide a suitable construction of a surgical handpiece for endocanalicular laser dacryocystorhinostomy capable of advancing the optical fibre fed through the handpiece in a stepwise manner so that the fibre can move for a specific, always equal distance towards the operation site every time its end projecting out of the cannula burns out due to high energy of the outgoing laser light. In addition, the construction of the handpiece shall ensure that the optical fibre is allowed to move exclusively in the direction towards the operation site, and that the fibre is set in motion only when pushed forward by the surgeon. Hence, it Is an important object of the handpiece construction that it does not allow the optical fibre to escape backwards into the cannula. The problem to be solved is how to move the optical fibre in one direction for more than one equally sized step. The handpiece shall further enable the surgeon to initiate the movement of the fibre in the course of the operation and to do this simply by using only one hand. Yet another required feature of the handpiece is to provide cooling of the tip of the optical fibre and of the treated tissue by irrigation with water running through the handpiece in such a manner that at the end of the handpiece the water flows out in drops or in a thin stream. Such washing out with water should at the same time facilitate dilation of the tear sac during the operation.

### Description of the related art

The human eye is continually flushed with tear fluid that provides moisture, protection and nourishment for the surface of the eye. Tears, which are produced in lacrimal glands located above and to the side of the eye and are spread over the surface of the eye by the blinking action of the eyelids, collect in the tear ducts and flow through the tear ducts into the lacrimal sac and from there into the nasal cavity. Due to various causes the passage between the lacrimal sac and the nasal cavity can be blocked. This condition can result in overflow of tears and in the event of bacterial infection also in painful inflammation and swelling of the lacrimal sac. In such case the passage can be re-established by a surgical operation called dacryocystorhinostomy or abbreviated DCR.

In long-established DCR procedure, first an incision is made by a scalpel beneath the eye above the lacrimal sac then the osteotomy is performed by cutting an opening in a nasal bone. Such operation is annoying and traumatic for the patient and leaves a scar on the face.

More advanced operation procedures are endonasal DCR and endoscopic DCR. To illuminate the lacrimal sac so that the site of the osteotomy can be determined, a light probe is inserted through the lacrimal duct. In the nose a piece of the mucous membrane and a small amount of bone are removed to permit a new connection between the lacrimal sac and the inside of the nose. For a certain time period two silicon tubes are inserted through the tear ducts, the lacrimal sac, and the opening to keep the newly created opening from scarring shut during healing. This operation is less unpleasant for the patient than the surgical operation performed on the face. However, it is rather time consuming and demanding for the surgeon.

Most recent DCR procedure is the endocanalicular laser dacryocystorhinostomy, such as described, for example, in patent US5345948 and in patent application US2007005120. The operation is performed from the outside of the eye. In order to allow the surgeon to monitor the operation and to precisely adjust the position of the optical fibre, an endoscope can be inserted into the nasal cavity. A cannula at the end of the handpiece, with an optical fibre inside that conducts a laser light emitted from a source of laser light suitable for tissue removal, is introduced into the lacrimal duct. As the fibre end burns out during the operation due to high energy of the laser light, the fibre has to be pushed towards the operation site to ensure that a fibre section of suitable length protrudes out of the cannula. The fibre end is cooled down during the operation by continual washing by water flowing through the cannula. Irrigation with water inhibits carbonization of tissue during the operation and consequently reduces the formation of scar tissue later on. At the end of the operative procedure, a bicanalicular intubation is performed by using silicone tubes. The endocanalicular DCR method enables the surgeon to perform the operative procedure much faster in comparison with the methods mentioned earlier. Other benefits of this procedure are as follows: there is no bleeding; only minor thermal injuries are inflicted to the tissue; there is very little swelling; the patient can leave the hospital the next day.

The major problem encountered in applying the latter method is how to control the length of the optical fibre section that protrudes out of the cannula. When the surgeon positions the cannula on the operation site, the fibre shall not protrude out of the cannula to avoid tissue injury. During the surgery procedure the end of the fibre burns out, so the end section of the fibre is very hot, therefore the fibre shall protrude out of the cannula all the time, otherwise the cannula would become hot as well and could cause thermal damage to the tissue. Consequently, the surgeon must advance the fibre through the cannula from time to time. To achieve a controlled movement of the fibre, the known solutions of the handpieces, such as described, for example, in DE19956516 and in DE102004007120, comprise a sliding device with engraved markings defining the individual incremental movements of the fibre. The sliding device can be moved in a continuous manner either in the direction towards the cannula or away from the cannula. The surgeon needs both his hands when adjusting the length of the fibre section that protrudes out of the cannula during the operation. The sliding device and with it the optical fibre can move in both directions, so it can easily happen, that in a momentary improvidence of the surgeon the end portion of the fibre slips back into the cannula or is pushed too far out of it. The surgeon may loose the feeling about the exact position of the fibre and is forced to interrupt the operation to push the fibre out for a desired distance. Such event may prolong the duration of the operation. It also creates additional burden for the surgeon and demands higher concentration.

Patent application WO2004/091380 A (SYNERGETICS INC [US]) from 28 October 2004 discloses a surgical handpiece suitable for endocanalicular laser dacryocystorhinostomy, comprising an actuating knob designed to trigger a controlled movement of an optical fibre, in the direction towards the operation site, said controlled movement being accomplished by means of a slider in which the optical fibre is fixed, whereby with each trigger action the optical fibre is moved out of the cannula disposed at the end of the handpiece for a step of precisely determined length. The present invention has different mechanism for moving the optical fibre in one direction for more than one equally sized step as solution described in WO2004/091380 A.

Patent application US 2002/1 51879 A1 (LOEB MARTIN P [US]) from 17 October 2002 describes a surgical handpiece with optical fibre having a ratcheting mechanism, using a toothed slider, which can be activated through a control button so that the fibre advances for a given distance. The present innovation has a control button in connection with a toothed slider, which has return spring and actuating knob for returning a control button in resting position.

### Description of the solution of the technical problem

The essential feature of the surgical handpiece according to the present invention relates to a controlled movement of the optical fibre intended for tissue removal, wherein the controlled movement is realized by means of a toothed slider in which the optical fibre is fixed and by means of an actuating knob designed to trigger the movement of the toothed slider and thereby also the movement of the optical fibre towards the operation site. With each trigger action the optical fibre moves out of the cannula disposed at the distal end of the handpiece for a step of precisely defined length. The construction of the handpiece is such that when the actuating knob is operated the optical fibre can travel only in the direction from the cannula towards the operation site, whereby the fibre moves forward only by one step with each activation of the actuating knob. The surgeon can push the fibre out of the cannula in the course of surgical operation, so there is no need to interrupt the operation procedure. Besides, it is not necessary for the surgeon to remember the position of the slider, since the information on the length of the fibre section that protrudes out of the cannula is provided by the toothed slider.

The present invention is defined in independent claim 1. Preferred embodiments are defined by the claims dependent thereon.

In the following detail description the surgical handpiece for endocanalicular laser DCR will be presented with reference to the accompanying figures showing:
- Figure 1:: a schematic depiction of the system for endocanalicular DCR with the surgical handpiece according to the present invention.
- Figure 2:: a schematic depiction of the shifting mechanism for moving the optical fibre shown in non-activated position.
- Figure 3:: a schematic depiction of the shifting mechanism for moving the optical fibre shown in activated position.

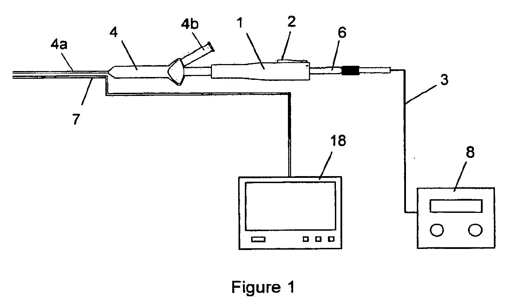

Figure 1 shows the entire system for endocanalicular DCR with the surgical handpiece according to the present invention. The optical fibre 3 is fed from the laser light source 8 through the handpiece and through the cannula disposed at the end of the handpiece. The surgical operation, that is the excavation and drilling of the tissue, is performed with the tip of the optical fibre 3 protruding beyond the thin-wall tube 4a of the cannula 4. The water for washing out the optical fibre end as well as the treated tissue runs through the tube 4b of the cannula 4.

The source 8 produces the laser light with wavelength of 980 nm. Sources of laser light with other wavelengths can be applied as well. As the end of the optical fibre 3 burns out during the operation, from time to time the fibre must be pushed out of the cannula 4 by means of the actuating knob 2. The surgeon may observe the length of the optical fibre section protruding out of the cannula on the monitor 18 by using the endoscope 7,

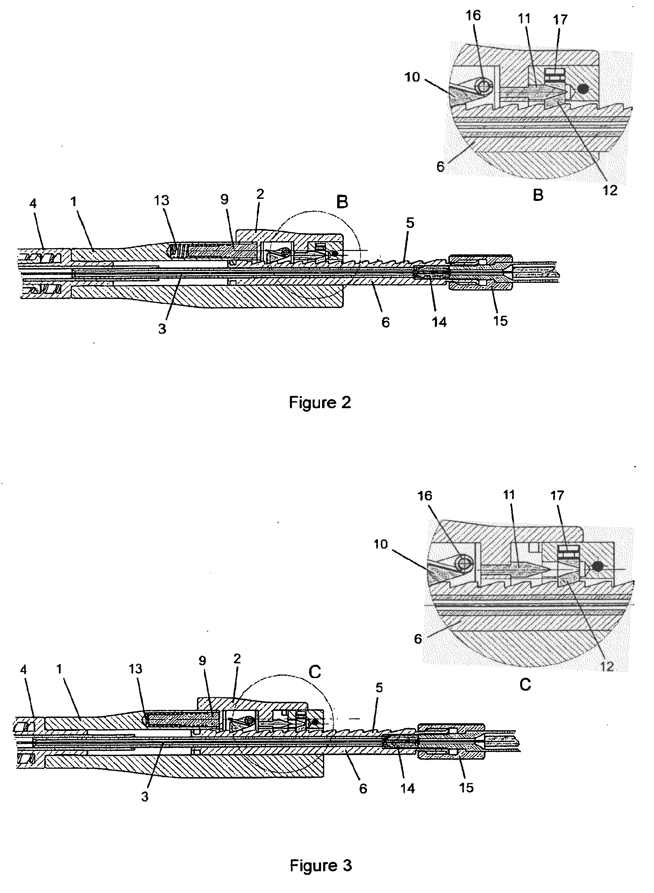

In Figure 2 a longitudinal section of the surgical handpiece is shown. The shifting mechanism for stepwise movement of the optical fibre 3 through the handpiece and through the cannula 4 towards the operation site is in non-activated position. The actuating knob 2 designed to slide along the surface of the handpiece body 1 is in abutment with the piston 9 pushed by the return spring 13 against the abutment with the actuating knob 2. The optical fibre 3 fed in the toothed slider 6 is fastened to the slider by a silicon clamp 14 and a ribbed ring 15 so that the optical fibre 3 is moved together with the toothed slider 6. The toothed slider 6 has serrated teeth 5 arranged on its upper side. The spring 16 fitted in the actuating knob 2 holds the spring tongue 10 engaged in one of the notches between two adjacent serrated teeth. The actuating knob 2 comprises a wedge-like member 11 engaged in the locking ring 12. When the actuating knob 2 is in non-activated position, the locking ring 12 is fixed tightly and cannot move either horizontally or vertically. The movement of the locking ring 12 in horizontal direction is prevented by serrated teeth 5 arranged on the slider 6, while the movement in the vertical direction is prevented by the wedge-like member 11. Consequently, with the actuating knob 2 in non-activated position the locking ring 12 holds the toothed slider 6 along with the optical fibre 3 in fixed position.

In Figure 3, the shifting mechanism for stepwise movement of the optical fibre 3 through the handpiece is shown in activated position. When the surgeon pushes the actuating knob 2 the spring tongue 10 and the wedge-like member 11 move along with the knob 2. The actuating knob 2 pushes also the piston 9 that compresses the return spring 13. The spring tongue 10 exerts pressure on the front slope of the notch between two teeth, where the said tongue 10 is engaged, trying to move the slider 6. The shifting of the slider 6 for one step is ultimately accomplished by means of the locking ring 12 as soon as the movement of the ring 12 in vertical direction is allowed which occurs when the cone formed wedge-like member 11 moves for a sufficient distance in the direction of movement of the actuating knob 2. The movement of the locking ring 12 in the vertical direction is instigated also by the form of the serrated teeth 5. The surgeon releases the actuating knob 2 when the knob reaches the uttermost end of its movement range.

As a result, the return spring 13 which has been compressed until this moment is released to urge through the piston 9 the actuating knob 2 along with the spring tongue 10 and the wedge-like member 11 back to resting position shown in Figure 2. The locking ring 12 which jumps over one tooth during movement of the toothed slider 6 is then forced by the pushing spring 17 to fall down into the notch behind the jumped tooth, so any further movement of the slider 6 is blocked. The spring tongue 10 resumes its resting position shown in Figure 2 after sliding smoothly over one tooth and snapping into the notch behind this tooth owing to a suitable form of serrated teeth 5. When the wedge-like member 11 returns to its previous position, it prevents further movement of the locking ring 12 as shown in Figure 2.

After release of the actuating knob 2, all the parts of the shifting mechanism, that is, the piston 9 with the return spring 13, the wedge-like member 11 and the spring tongue 10 with the spring 16, and the locking ring 12 with the compression spring 17, return to their previous positions as shown in Figure 2, except for the slider 6 which advances towards the cannula 4 by one tooth pitch. Along with the slider 6, the optical fibre 3 fixed at the entry into the slider 6 moves for an equal distance and in the same direction.

The shifting mechanism does not allow the optical fibre 3 to move in the opposite direction, that is, in the direction from the operation site to the cannula 4. In this way escaping of the fibre back into the cannula is prevented to avoid overheating of the cannula that could cause permanent injury to the tissue.

The surgeon can put the actuating knob and the shifting mechanism in motion simply by a finger of the hand holding the handpiece. As with a single activation of the actuating knob the optical fibre is shifted for a known and always the same distance, an endoscope for monitoring and adjusting the length of the fibre section that extends out of the cannula is in fact not required. So, besides lesser burden for the surgeon and higher safety for the patient, the handpiece according to the invention can provide cost reduction as well.

## Claims

1. A surgical handpiece for endocanalicular laser dacryocystorhinostomy, comprising
- a handpiece body with an actuating knob (2), said actuating knob (2) being designed to trigger a controlled step-by-step movement of a optical fibre (3), fed from a laser light source (8), only in the direction towards the operation site, said controlled movement being accomplished by means of a toothed slider (6) in which the optical fibre (3) is fixed, whereby with each trigger action the optical fibre (3) is moved out of a cannula (4) inserted in the handpiece body, disposed at the end of the handpiece and designed to lead the optical fibre (3) to the operation site, for only one step of precisely determined length, the toothed slider (6) allowing for movement of the optical fibre (3) in one direction for more than one equally sized step;
- the toothed slider (6), which is mounted in the handpiece body along the longitudinal axis of the body and has a row of serrated teeth (5) disposed on its upper side, having a hollow interior in which the optical fibre (3) is fed along the longitudinal axis of the slider, the optical fibre (3) being fixed to the slider (6) at the entry into the slider (6);
the surgical handpiece additionally comprising
- a shifting mechanism designed to execute a stepwise advancement of the optical fibre (3), fed through the handpiece, for a precisely determined step length towards the operation site, wherein said advancement is initiatable through a single manipulation of the actuating knob (2) each time the end of the optical fibre section, which projects out of the cannula (4) and with which the surgical operation is performed, burns out due to high energy of the outgoing laser light,
- said shifting mechanism including a wedge-like member (11) and a spring tongue (10) with a spring (16), said wedge-like member (11) and spring tongue (10) being incorporated in the actuating knob (2) wherein their task is to push the actuating knob (2) in the direction towards the operation site;
- said shifting mechanism further including the row of serrated teeth (5), said serrated teeth (5) being disposed on the upper side of the slider (6), wherein their task is to ensure stepwise shifting of the toothed slider (6);
- said shifting mechanism further including a return spring (13) with a piston (9), said return spring (13) being compressed when the actuating knob (2) is pushed towards the cannula (4) and expands once the pushing action on the actuating knob (2) ceases, wherein the stretched spring (13) pushes by means of the piston (9) the actuating knob (2) along with the spring tongue (10) and the wedge-like member (11) in backward direction, so that they all resume their original positions;
- said shifting mechanism further including a locking ring (12) with a pushing spring (17), said locking ring (12) blocking the movement of the toothed slider (6) when the actuating knob (2) is in a resting state.

2. The surgical handpiece according to claim 1, **characterized in that** the wedge-like member (11), which is engaged in the locking ring (12) during the resting state of the actuating knob (2), moves out of the locking ring (12) when the actuating knob (2) is pushed to move the fibre (3) in the desired direction, thereby allowing the locking ring (12) to get out of the notch between two teeth so that the movement of the toothed slider (6) is no longer blocked by the locking ring (12) and that the spring tongue (10), which from the moment the surgeon begins to push the actuating knob (2) exerts pressure on the toothed slider (6) in the desired direction of movement of the fibre, can finely accomplish the movement of the toothed slider (6).

3. The surgical handpiece according to claims 1 and 2, **characterized in that** during the movement of the toothed slider (6) the locking ring (12) jumps only over one tooth in the row of teeth (5) as due to the operation of the pushing spring (17), which presses the locking ring (12) down, the locking ring (12) falls into the notch following the jumped tooth and thereby prevents any further movement of the toothed slider (6).

4. The surgical handpiece according to claims 1 to 3, **characterized in that** a release of the actuating knob (2), that occurs when the actuating knob (2) reaches the uttermost end position of its movement range in the direction towards the operation site, releases the return spring (13) and through the piston (9) urges the actuating knob (2) along with the spring tongue (10) and the wedge-like member (11) back to resting position.

5. The surgical handpiece according to claims 1 to 4, **characterized in that** the teeth in the row of teeth (5) have a serrated form, wherein the sloping sides of the teeth are facing the cannula (4), so when the actuating knob (2) returns to its resting position, the spring tongue (10) slides easily over one tooth and locks into the notch behind said tooth.

6. The surgical handpiece according to claims 1 to 5, **characterized in that** the handpiece enables irrigation with water around the end of the optical fibre during operation.

## Patentansprüche

1. Chirurgisches Handstück für endokanalikuläre Laser-Dakryozystorhinostomie umfassend
- ein Handstück-Körper mit einem Betätigungsknopf (2), wobei der genannte Betätigungsknopf (2) zum Auslösen einer gesteuerten Schrittbewegung einer von einer Laserlichtquelle (8) zugeführten optischen Faser ausgelegt ist, nur in Richtung zur Operationsstelle, wobei die genannte gesteuerte Bewegung mit Hilfe eines Zahnschiebers (6), in welchem die optische Faser (3) fixiert ist, ausgeführt wird, womit sich bei jeder Betätigung die optische Faser (3) aus der im Handstück-Körper eingefügten, am Ende des Handstücks angeordneten und zur Führung der optischen Faser (3) zur Operationsstelle ausgelegten Kanüle (4) bewegt, für nur einen Schritt einer genau definierten Länge, wobei der Zahnschieber (6) die Bewegung der optischen Faser (3) in einer Richtung für mehr als einen Schritt der gleichen Länge ermöglicht;
- den Zahnschieber (6), der im Handstück-Körper entlang der Längsachse des Körpers gelagert ist und eine an seiner Oberseite angeordneten Reihe von gezackten Zähnen (5) aufweist, der einen hohlen Innenraum, in welchem die optische Faser (3) entlang der Längsachse des Schiebers geführt wird, aufweist, wobei die optische Faser (3) am Schieber (6) am Eingang zum Schieber (6) fixiert ist;
wobei das chirurgische Handstück des Weiteren umfasst
- einen zum Ausführen der schrittweisen Fortbewegung der durch das Handstück zugeführten optischen Faser (3) ausgelegten Schiebemechanismus für eine genau vorbestimmte Schrittlänge zur Operationsstelle, wobei die genannte Fortbewegung durch eine einzelne Betätigung des Betätigungsknopfes (2) jeweils am Ende des Abschnitts der optischen Faser betätigt wird, wenn der aus der Kanüle (4) herausragender Abschnitt, mit welchem die chirurgische Operation ausgeführt wird, wegen der hohen Energie des austretenden Laserlichts ausbrennt,
- wobei der genannte Schiebemechanismus ein keilförmiges Element (11) und eine Federzunge (10) mit einer Feder (16) aufweist, wobei das keilförmige Element (11) und die Federzunge (10) im Betätigungsknopf enthalten sind und zur Aufgabe haben, den Betätigungsknopf (2) in Richtung hin zur Operationsstelle zu schieben;
- wobei der Schiebemechanismus des Weiteren eine Reihe von gezackten Zähnen (5) aufweist, die an der Oberseite des Schiebers (6) angeordnet sind und deren Aufgabe eine schrittweise Fortführung des Zahnschiebers (6) ist;
- wobei der Schiebemechanismus des Weiteren eine Rückstellfeder (13) mit einem Kolben (9) umfasst, wobei die genannte Rückstellfeder (13) zusammengedrückt wird, wenn der Betätigungsknopf (2) zur Kanüle (4) geschoben wird, und entlastet wird, wenn die Drucktätigkeit des Betätigungsknopfs (2) nachlässt, wobei die entlastete Feder (13) mit Hilfe des Kolbens (9) den Betätigungsknopf (2) samt Federzunge (10) und den keilförmigen Elementen (11) in Rückwärtsrichtung drückt, sodass alle ihre Ausgangstellungen einnehmen;
- wobei der Schiebemechanismus des Weiteren einen Klemmring (12) mit einer Zubringerfeder (17) aufweist, wobei der Klemmring (12) die Bewegung des Zahnschiebers (6) blockiert, wenn sich der Betätigungsknopf (2) in Ruhestellung befindet.

2. Chirurgisches Handstück nach Anspruch 1, **dadurch gekennzeichnet, dass** das im Ruhestand des Betätigungsknopfs (2) sich im Klemmring (12) befindliche keilförmige Element (11) aus dem Klemmring (12) austritt, wenn der Betätigungsring (2) betätigt wird, um die Faser (3) in einer gewünschten Richtung zu bewegen, womit der Klemmring (12) aus der Nut zwischen zwei Zähnen austreten kann, sodass die Bewegung des Zahnschiebers (6) nicht mehr durch den Klemmring (12) blockiert ist und dass die Federzunge (10), die, sobald der Chirurg den Betätigungsknopf (2) andrückt, den Druck auf den Zahnschieber (6) in der gewünschten Richtung der Faser ausübt, die Bewegung des Zahnschiebers (6) genau beenden kann.

3. Chirurgisches Handstück nach Ansprüchen 1 und 2, **dadurch gekennzeichnet, dass** während der Bewegung des Zahnschiebers (6) der Klemmring (12) nur über einen Zahn in der Reihe von Zähnen (5) springen kann, da wegen der Tätigkeit der Zubringerfeder (17), die den Klemmring (12) nach unten drückt, der Klemmring (12) in die, den übersprungenen Zahn folgenden Nut einrastet und damit jede weitere Bewegung des Zahnschiebers (6) verhindert.

4. Chirurgisches Handstück nach Ansprüchen 1 bis 3, **dadurch gekennzeichnet, dass** das Auslösen des Betätigungsknopfs (2), das dann eintritt, wenn der Betätigungsknopf (2) die äußerste Endstellung seines Bewegungsbereichs in Richtung hin zur Operationsstelle annimmt, die Rückstellfeder (13) auslöst und mit dem Kolben (9) den Betätigungsknopf (2) samt Federzunge (10) und den keilförmigen Elementen (11) zurück in die Ruhestellung bringt.

5. Chirurgisches Handstück nach Ansprüchen 1 bis 4, **dadurch gekennzeichnet, dass** die Zähne in der Reihe von Zähnen (5) eine gezackte Form aufweisen, wobei die geneigten Seiten der Zähne der Kanüle (4) zugewandt sind, also wenn der Betätigungsknopf (2) in die Ruhestellung zurückkehrt, die Federzunge (10) einfach über einen Zahn gleitet und in die Nut hinter dem genannten Zahn einrastet.

6. Chirurgisches Handstück nach Ansprüchen 1 bis 5, **dadurch gekennzeichnet, dass** das Handstück eine Spülung mit Wasser um der Faserspitze während der Operation ermöglicht.

## Revendications

1. Une pièce à main chirurgicale pour dacryocystorhinostomie au laser par voie endocanaliculaire comprenant
- un corps de la pièce à main chirurgicale avec un bouton d'actionnement (2), ledit bouton d'actionnement (2) étant conçu pour déclencher le mouvement pas à pas contrôlé d'une fibre optique (3), alimentée par une source de lumière laser (8), seulement en direction du site d'opération, ledit mouvement contrôlé se réalisant au moyen d'un coulisseau denté (6), dans lequel la fibre optique (3) est fixée, la fibre optique (3) se déplaçant, à chaque action de déclenchement, hors de la canule (4) insérée dans le corps de la pièce à main, disposée à l'extrémité de la pièce à main et conçue pour conduire la fibre optique (3) vers le site d'opération, pour seulement un pas d'une longueur précisément déterminée, le coulisseau denté (6) permettant le mouvement de la fibre optique (3) dans une direction pour plus d'un pas d'une taille égale;
- le coulisseau denté (6) étant agencé dans le corps de la pièce à main le long de l'axe longitudinal du corps et pourvu d'une rangée de dents dentelées (5) disposées sur le côté supérieur, ayant un intérieur creux dans lequel la fibre optique (3) est introduite le long de l'axe longitudinal du coulisseau, la fibre optique (3) étant fixée au coulisseau (6) au niveau de l'entrée du coulisseau (6);
la pièce à main chirurgicale comprenant en outre
- un mécanisme de déplacement conçu pour réaliser un avancement pas à pas de la fibre optique (3) introduite à travers la pièce à main, pour une longueur de pas précisément prédéterminée vers le site d'opération, ledit avancement s'initiant par une seule manipulation du bouton d'actionnement (2) à chaque fois que l'extrémité de la section de la fibre optique qui fait saillie hors de la canule (4) et avec laquelle l'opération chirurgicale est effectuée brûle entièrement en raison de l'énergie élevée de la lumière laser émise,
- ledit mécanisme de déplacement incluant un élément en forme de coin (11) et une languette élastique (10) avec un ressort (16), ledit élément en forme de coin (11) et la languette élastique (10) étant incorporés dans le bouton d'actionnement (2), leur tâche étant de pousser le bouton d'actionnement (2) dans la direction du site d'opération;
- ledit mécanisme de déplacement incluant en outre une rangée de dents dentelées (5), lesdites dents dentelées (5) étant disposées sur le côté supérieur du coulisseau (6), leur tâche étant d'assurer le mouvement pas à pas du coulisseau denté (6);
- ledit mécanisme de déplacement incluant en outre un ressort de rappel (13) avec un piston (9), ledit ressort de rappel (13) étant compressé quand le bouton d'actionnement (2) est poussé vers la canule (4) et se détendant lorsque cesse la poussée sur le bouton d'actionnement (2), le ressort détendu (13) poussant au moyen du piston (9) le bouton d'actionnement (2) avec la languette élastique (10) et avec l'élément en forme de coin (11) vers l'arrière, pour que tous reprennent leurs positions initiales;
- ledit mécanisme de déplacement comprenant en outre un anneau de verrouillage (12) avec un ressort de poussée (17), ledit anneau de verrouillage (12) bloquant le mouvement du coulisseau (6) denté lorsque le bouton d'actionnement (2) en état de repos.

2. La pièce à main chirurgicale selon la revendication 1, **caractérisée en ce que** l'élément en forme de coin (11), qui est engagé dans l'anneau de verrouillage (12) pendant que le bouton d'actionnement (2) est en état de repos, se déplace hors de l'anneau de verrouillage (12) quand le bouton d'actionnement (2) est poussé pour déplacer la fibre (2) dans la direction souhaitée, permettant ainsi à l'anneau de verrouillage (12) de sortir de la rainure entre deux dents, de sorte que le mouvement du coulisseau denté (6) n'est plus bloqué par l'anneau de verrouillage (12) et que la languette élastique (10), qui exerce une pression sur le coulisseau denté (6) dans la direction souhaitée du mouvement de la fibre dès que le chirurgien commence à appuyer sur le bouton d'actionnement (2), peut accomplir le mouvement du coulisseau denté (6).

3. La pièce à main chirurgicale selon les revendications 1 et 2, **caractérisée en ce que** pendant le mouvement du coulisseau denté (6), l'anneau de verrouillage (12) enjambe seulement une dent dans la rangée de dents (5), parce que, en raison de l'action du ressort de poussée (17) qui pousse l'anneau de verrouillage (12) vers le bas, l'anneau de verrouillage (12) tombe dans la rainure qui suit la dent enjambée, prévenant ainsi tout mouvement supplémentaire du coulisseau denté (6).

4. La pièce à main chirurgicale selon les revendications 1 à 3, **caractérisée en ce que** le relâchement du bouton d'actionnement (2), qui survient lorsque le bouton d'actionnement (2) atteint la position extrême de sa zone de mouvement dans la direction du site d'opération, relâche le ressort de rappel (13) et force, par l'intermédiaire du piston (9), le bouton d'actionnement (2) avec la languette élastique (10) et l'élément en forme de coin (11) à se remettre en position de repos.

5. La pièce à main chirurgicale selon les revendications 1 à 4, **caractérisée en ce que** les dents dans la rangée de dents (5) ont une forme dentelée, les côtés inclinés des dents étant tournés vers la canule (4), de sorte que lorsque le bouton d'actionnement (2) retourne en position de repos, la languette élastique (10) glisse facilement par-dessus une dent et se verrouille dans la rainure derrière ladite dent.

6. La pièce à main chirurgicale selon les revendications 1 à 5, **caractérisée en ce que** la pièce à main permet l'irrigation avec de l'eau autour de l'extrémité de la fibre optique pendant l'opération.
